# EUROPÄISCHE PATENTSCHRIFT

(11) **EP 0 986 326 B1**
(45) Veröffentlichungstag und Bekanntmachung des Hinweises auf die Patenterteilung: **01.06.2005**
(21) Anmeldenummer: 98931946.2
(22) Anmeldetag: 18.04.1998
(51) Int. Cl.: A61B 5/103, A61B 5/00, G01N 21/47

(54) **In-vivo Detektion der Richtung von Linien einer kosmetisch optimalen chirurgischen Schnittführung**
In vivo detection of lines representing the direction of a cosmetically optimal surgical cut
Détection in vivo de lignes représantatives de la direction d'une incision esthètiquement optimale

(30) Priorität: 26.05.1997 DE 19721902
(43) Veröffentlichungstag der Anmeldung: 22.03.2000
(73) Patentinhaber: Nickell, Stephan, 69117 Heidelberg (DE); Essenpreis, Matthias, 82131 Gauting (DE); Boecker, Dirk, 69115 Heidelberg (DE)
(72) Erfinder: Nickell, Stephan, 69117 Heidelberg (DE); Essenpreis, Matthias, 82131 Gauting (DE); Boecker, Dirk, 69115 Heidelberg (DE)
(74) Vertreter: Pfeifer, Hans-Peter, Dr.
(86) Internationale Anmeldenummer: PCT/DE1998/001090
(87) Internationale Veröffentlichungsnummer: WO 1998/053739

(56) Entgegenhaltungen:
- EP-A- 0 777 119
- WO-A-94/10901
- DE-A- 4 303 047
- DE-A- 4 331 010
- DE-A- 4 427 101
- DE-U- 9 308 617
- US-A- 4 398 541

## Beschreibung

Grundlegende Untersuchungen über die kosmetisch optimale chirurgische Schnittführung wurden von K. Langer bereits 1861 publiziert. Er erzeugte in der Haut von Leichen Stichwunden in engem Abstand. Obwohl das Stichinstrument rund war, entstanden ovale Löcher. Die Längsachsen dieser Wunden bilden einen regelmäßigen Verlauf, der nach dem Namen ihres Entdeckers als Langer-Linien bezeichnet wird.

Ein chirurgischer Schnitt sollte eine optimale Freilegung des Operationsgebietes und eventuell eine intraoperative Erweiterungsmöglichkeit bieten. Zugleich soll sichergestellt sein, daß nach der Abheilung ein kosmetisch möglichst günstiges Ergebnis erzielt wird. Diese Bedingungen werden im allgemeinen optimal erfüllt, wenn der Schnitt in der Richtung bestimmter Linien geführt wird. Insbesondere wird dabei die Narbenbildung minimiert. Dies ist besonders wichtig in der plastischen Chirurgie, wo die Operationsnarben oft an gut sichtbaren Körperstellen, insbesondere im Gesicht, verlaufen.

In dem Artikel "Relaxed Skin Tension Lines (RSTL) versus other Skin Lines" von A.F. Borges, "Plastic and reconstructive surgery", 1984, 144 bis 150 werden aus medizinischer Sicht verschiedene Verfahren zur Ermittlung der für eine optimale chirurgische Schnittführung geeigneten Linien gegenübergestellt. Es wird dargelegt, daß von verschiedenen Autoren ganz unterschiedliche physikalische, anatomische, funktionale oder empirische Methoden vorgeschlagen wurden, um dieses Problem zu lösen. Hinsichtlich des ursprünglichen Verfahrens von Langer kommt der Autor zu dem Ergebnis, daß die an Leichen ermittelten Hautspaltlinien durch die Totenstarre verfälscht sind und in der Mehrzahl der Fälle keine richtigen Ergebnisse liefern. Dennoch wurden die für die optimale chirurgische Schnittführung empfohlenen Linien von späteren Autoren vielfach als "Langer-Linien" bezeichnet, auch wenn sie nicht nach dem Langer-Verfahren ermittelt wurden. Der Autor verwendet statt dessen synonym die Begriffe "lines of tension" oder "cleavage lines".

Wie dieses Beispiel zeigt, werden die Linien der optimalen Schnittführung in der Literatur uneinheitlich bezeichnet, wobei u.a. die Begriffe "Langer-Linien" oder "Hautspaltlinien" (Sulci cutis) verwendet werden. Nachfolgend wird als Kurzbezeichnung der Begriff "optimale Schnittführungslinien" verwendet.

Bedauerlicherweise ist der genaue Verlauf der optimalen Schnittführungslinien in vielen Fällen nicht bekannt. In einigen Körperpartien bestehen große Unterschiede von Mensch zu Mensch. Auch bei derselben Person können sich im Laufe des Lebens Veränderungen ergeben. Deshalb wäre es notwendig, die optimalen Schnittführungslinien in vivo mit einem nichtinvasiven Verfahren detektieren zu können. Damit wäre es selbst weniger erfahrenen Chirurgen möglich, eine Operation mit minimierter Narbenbildung planen zu können. Es würden nicht nur die mit starken Narben verbundenen psychischen und ästhetischen Probleme behoben, sondern auch der oft mit einer nachoperativen Narbenbehandlung verbundene Aufwand vermieden.

Trotz der großen Bedeutung dieses Problems ist ein geeignetes Verfahren bisher nicht verfügbar. Versucht wurden insbesondere mechanische Spannungs-Dehnungs-Messungen an der Hautoberfläche, wie sie beispielsweise von J. C. Barbenell in dem Beitrag "Identification of Langer's-Lines", pp. 341-344 in: Handbook of Non-Invasive Methods and the Skin, CRC Press, Boca Raton, 1995, beschrieben sind. In der Publikation wird deutlich, daß mit den diskutierten Verfahren keine hinreichend verläßliche Information über die Orientierung der optimalen Schnittführungslinien in der Haut gewonnen werden kann.

Der Erfindung liegt die Aufgabe zugrunde, die Richtung des Verlaufes der optimalen Schnittführungslinien an der Hautoberfläche in vivo nichtinvasiv und schmerzfrei zu detektieren.

Die Aufgabe wird erfindungsgemäß mit Hilfe von Licht gelöst. Primärlicht wird an einer punktförmigen Einstrahlungsstelle lokalisiert derartig durch die Hautoberfläche eingestrahlt, daß das Licht in der Haut in Abhängigkeit von der Streuung und der optischen Absorption transportiert wird. Ein Teil des eingestrahlten Lichtes tritt dabei als Sekundärlicht in der Umgebung der Einstrahlungsstelle wieder aus. Als Maß für die Richtung der optimalen Schnittführungslinien wird die Polarwinkel-Abhängigkeit einer meßbaren Eigenschaft des aus der Haut austretenden Sekundärlichtes um die Einstrahlungsstelle herum gemessen und eine Vorzugsrichtung des diffusen Lichttransportes in der Haut bestimmt.

Die meßbare Eigenschaft des Lichts (nachfolgend auch: "Meßgröße") ist insbesondere seine Intensität. Es können jedoch auch andere meßbare Eigenschaften verwendet werden, um die Vorzugsrichtung des diffusen Lichttransports in der Haut zu bestimmen. Beispielsweise kann das Primärlicht itensitätsmoduliert eingestrahlt werden. In diesem Fall kann die AC-Amplitude (Modulationsgrad) des detektierten Sekundärlichts eine Information über die Vorzugsrichtung des diffusen Lichttransports geben. Bei Einstrahlung von polarisiertem Primärlicht kommt als weiteres Beispiel der Polarisationsgrad des Sekundärlichts in Betracht. Geeignet ist ganz allgemein jede meßbare Eigenschaft des Sekundärlichts, die eine Information über eine Vorzugsrichtung des diffusen Lichttransports enthält. Nachfolgend wird ohne Beschränkung der Allgemeinheit beispielhaft auf die Intensität als Meßgröße Bezug genommen.

Zur Ermittlung der Polarwinkel-Abhängigkeit wird das Primärlicht an einer kleinen, räumlich begrenzten Einstrahlungsstelle in die Haut eingestrahlt. Die Messung der Polarwinkel-Abhängigkeit um die Einstrahlungsstelle herum setzt voraus, daß die Intensität des aus der Haut austretenden Sekundärlichtes an mindestens zwei Detektionsstellen gemessen wird, deren Polarwinkel-Richtungen, bezogen auf die Einstrahlungsstelle, eine orthogonale Komponente haben. Wenn nur zwei Detektionsstellen vorgesehen sind, sollten sie vorzugsweise in einem Polarwinkel von etwa 90° zueinander angeordnet sein, wobei der Polarwinkel-Abstand zumindest größer als 35° sein sollte.

Um eine gute Auflösung der gemessenen Polarwinkel-Abhängigkeit der Meßgröße zu erreichen, sollten mindestens drei Meßstellen vorgesehen sein, die zueinander jeweils (paarweise) einen Polarwinkel-Abstand von weniger als 20° haben. Wesentlich bevorzugt ist es, die Meßgröße an einer Vielzahl von rings um die Einstrahlungsstelle angeordneten Detektionsstellen zu bestimmen, wobei eine Polarwinkel-Auflösung von mindestens etwa 20° vorteilhaft ist, d.h. der Polarwinkel-Abstand von Meßstelle zu Meßstelle sollte höchstens 20° betragen.

Die Wellenlänge des Primärlichts sollte bevorzugt zwischen 400 nm und 1400 nm liegen, wobei es im Hinblick auf die Meßgenauigkeit weiterhin vorteilhaft ist, wenn das Licht im wesentlichen monochromatisch ist. Dabei sind die Anforderungen an eine schmale Bandbreite allerdings gering. Es genügt, wenn die Halbwertsbreite des Primärlichts kleiner als 200 nm, bevorzugt kleiner als 100 nm ist. Weiterhin kann es für eine genaue Bestimmung der Vorzugsrichtung und damit der Richtung der optimalen Schnittführungslinien vorteilhaft sein, wenn das eingestrahlte Primärlicht polarisiert ist. Unter Umständen kann es auch nützlich sein, auf dem Weg des Sekundärlichts zwischen der Detektionsstelle und dem Lichtempfänger einen als Analysator wirkenden Polarisationsfilter anzuordnen.

Unterschiedliche Detektionsstellen können grundsätzlich mit Hilfe beweglicher Detektionsmittel, beispielsweise mit Hilfe von Lichtleitfasern, realisiert werden. Wesentlich bevorzugt sind jedoch Ausführungsformen, bei denen die Polarwinkel-Abhängigkeit mittels einer Mehrzahl von Detektionsmitteln gemessen wird, welche in fester räumlicher Zuordnung zu der Einstrahlungsstelle angeordnet sind und jeweils die Intensität des an einer definierten Detektionsstelle austretenden Sekundärlichts erfassen.

Die Detektionsstellen, deren Meßwerte zur Ermittlung einer Polarwinkel-Abhängigkeit um eine Einstrahlungsstelle verwendet werden, sind bevorzugt auf einem Kreis um die Einstrahlungsstelle angeordnet, so daß sie gleichmäßige Abstände von dieser haben. Dadurch ist die mathematische Auswertung am einfachsten. Darüber hinaus hat sich gezeigt, daß in bestimmten Hautpartien die Anisotropie für unterschiedliche Abstände zwischen Einstrahlungsstelle und Detektionsstelle verschieden ist. Dies hängt damit zusammen, daß die mittlere Eindringtiefe des Lichts vom Abstand zwischen der Einstrahlungsstelle und der Detektionsstelle abhängt.

Die Messung der Polarwinkel-Abhängigkeit der Intensität kann unter Berücksichtigung von hier erläuterten Besonderheiten weitgehend mit Verfahren und Vorrichtungen erfolgen, die für andere Zwecke bekannt sind. So ist in der WO 94/10901 ein Verfahren zur Analyse von Glucose in der Haut beschrieben, bei dem das Analyseergebnis aus mehreren "ortsaufgelösten Streulichtmessungen" bestimmt wird. Bei diesen Messungen wird jeweils Licht in einem definierten Teilbereich der Hautoberfläche (Einstrahlungsort) eingestrahlt und die an einem anderen definierten Teilbereich der Hautoberfläche in der Umgebung des Einstrahlungsortes (Detektionsort) austretende Lichtintensität gemessen. Zur Durchführung dieser Messungen kann insbesondere eine schachbrettartige Anordnung von in die Haut einstrahlenden Lichtquellen (insbesondere Leuchtdioden) und Detektoren (insbesondere Photodioden) verwendet werden. Eine für derartige Messungen sowie auch für andere Lichttransportmessungen an der Haut besonders geeignete Vorrichtung, bei der die Lichtübertragung von der Hautoberfläche zu den Detektoren über eine auf der Haut aufliegende optische Faserplatte erfolgt, ist Gegenstand der europäischen Patentanmeldung mit der Publikationsnummer 0777119. Ergänzend zu der hier gegebenen Beschreibung wird auf diese Publikation Bezug genommen.

In den genannten Publikationen wird davon ausgegangen, daß die durch optische Absorption und Streuung bestimmte Lichtausbreitung in der Hautoberfläche in der Umgebung einer eng begrenzten Einstrahlungsstelle im wesentlichen isotrop ist. Störungen der isotropen Ausbreitung werden lediglich insoweit erwartet, wie sie durch Unregelmäßigkeiten in der Haut wie beispielsweise Pigmentkonzentrationen (Leberflecken) oder dicht unter der Hautoberfläche verlaufende Adern verursacht werden.

In zahlreichen Publikationen geht es darum, unter der Hautoberfläche liegende "Strukturen", insbesondere Tumoren, mit Hilfe von in die Haut eingestrahltem Licht zu detektieren. Dies wird insbesondere in der WO 96/04545 und den darin einleitend erörterten Literaturstellen diskutiert. Auch dabei wird davon ausgegangen, daß der Lichttransport in der Haut, abgesehen von der Störung durch die gesuchten Strukturen, isotrop ist.

Abweichend von den Annahmen dieser Publikation wurde im Rahmen der vorliegenden Erfindung festgestellt, daß sich das Licht unter den hier beschriebenen Meßbedingungen in einem erstaunlich deutlichen Maß anisotrop ausbreitet und diese Anisotropie der Richtung des Verlaufs der Linien der optimalen chirurgischen Schnittführung entspricht.

Die Erfindung wird im folgenden anhand von in den Figuren schematisch dargestellten Ausführungsbeispielen näher erläutert.

Es zeigen:
- Fig. 1: ein Gerät zur Detektion der Richtung von optimalen Schnittführungslinien in perspektivischer Seitenansicht,
- Fig. 2: einen Schnitt durch das Gerät gemäß Fig. 1, wobei die Elektronik als Blockschaltbild dargestellt ist,
- Fig. 3: eine Detaildarstellung des Hautkontaktteils des in Fig. 2 dargestellten Geräts,
- Fig. 4: eine Aufsicht auf die untere Oberfläche einer Lichtempfängereinheit entlang der Linie IV-V in Fig. 3.
- Fig. 5: die Anordnung der Einstrahlungs- und Detektionsstellen bei einer alternativen Ausführungsform einer Lichtempfängereinheit,
- Fig. 6: eine Prinzipdarstellung zur Verdeutlichung der Verwendung des erfindungsgemäßen Geräts im Bauchbereich,
- Fig. 7: eine Darstellung des Verlaufs der optimalen Schnittführungslinien in dem gleichen Bereich,
- Fig. 8: ein Polarwinkel-Diagramm der Intensitätsverteilung von aus der Haut in der Umgebung einer punktförmigen Einstrahlungsstelle austretendem Licht bei einer Messung an der Bauchdecke, wie in den Fig. 6 und 7 dargestellt,
- Fig. 9: ein Polarwinkel-Diagramm wie Fig. 8, jedoch für zwei verschiedene Meßabstände zwischen der Einstrahlungsstelle und den Detektionsstellen,
- Fig. 10: eine Prinzipdarstellung einer alternativen Ausführungsform mit Darstellung des Verlaufs der optimalen Schnittführungslinien mittels einer Laser-Projektion.

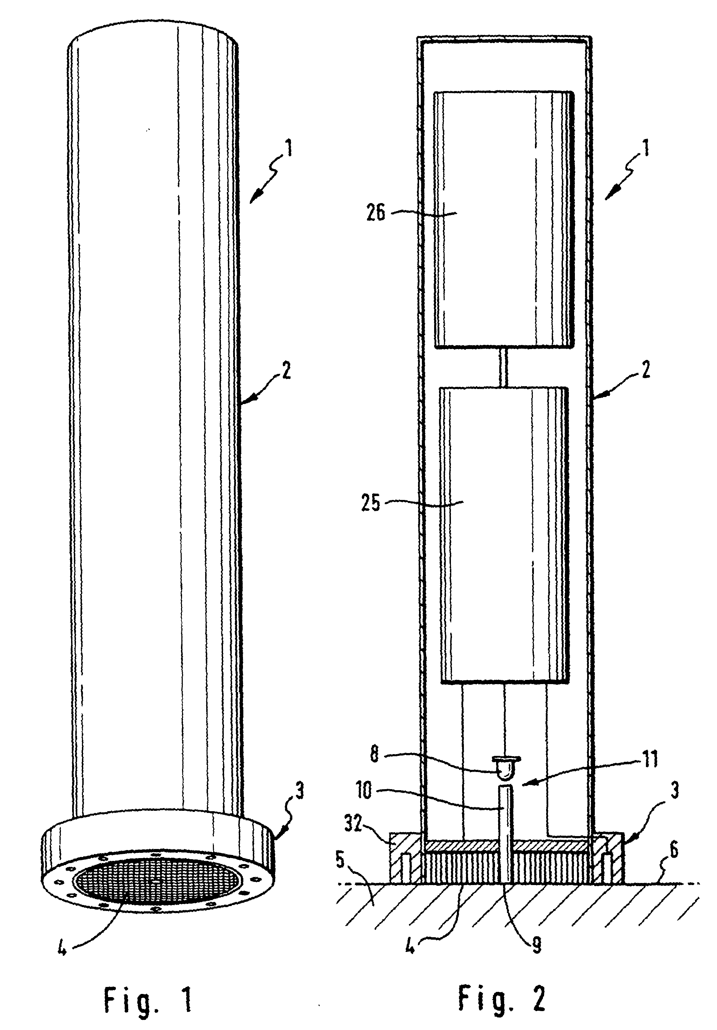

Das in den Figuren 1 bis 4 dargestellte Gerät 1 zur Detektion von optimalen Schnittführungslinien ist stabförmig ausgebildet, um eine leichte und präzise Führung zu ermöglichen. Es läßt sich in einen zylinderförmigen Oberteil 2 und einen Hautkontaktteil 3 unterteilen, dessen untere Begrenzungsfläche bei der Benutzung auf die Haut 5 aufgelegt wird und deswegen als Kontaktfläche 4 bezeichnet wird.

Mittels eines Lichtsenders 8, der insbesondere als Leuchtdiode oder Laserdiode ausgebildet sein kann, wird Primärlicht durch die Hautoberfläche 6 in die Haut 5 eingestrahlt. Die Lichtpassagestelle 9, an der das Primärlicht die Kontaktfläche 4 durchdringt, befindet sich im Zentrum der Kontaktfläche 4. Ihre Form und Größe definieren die Dimensionen einer punktförmigen Einstrahlungsstelle 7 an der Oberfläche 6 der Haut 5. Bevorzugt ist ihr Durchmesser kleiner als 1 mm, besonders bevorzugt kleiner als 0,5 mm. Bei der dargestellten bevorzugten Ausführungsform wird eine optische Verbindung zwischen dem Lichtsender 8 und der Lichtpassagestelle 9 durch einen Lichtleiter 10 hergestellt. Die Elemente, die zum Einstrahlen des Lichts an einer Einstrahlungsstelle dienen (hier Lichtsender 8 und Lichtleiter 10), werden insgesamt als Einstrahlungsmittel 11 bezeichnet.

Um die Polarwinkel-Abhängigkeit der Intensität des aus der Haut 5 in der Umgebung der Einstrahlungsstelle 7 austretenden Lichts zu detektieren, sind insgesamt mit 14 bezeichnete Detektionsmittel vorgesehen, von denen jedes so ausgebildet ist, daß die Intensität des Lichts jeweils an einer definierten Detektionsstelle 15 gemessen wird, die (ebenso wie die Einstrahlungsstelle 7) einen definierten, räumlich begrenzten Teilbereich der Hautoberfläche 6 bildet.

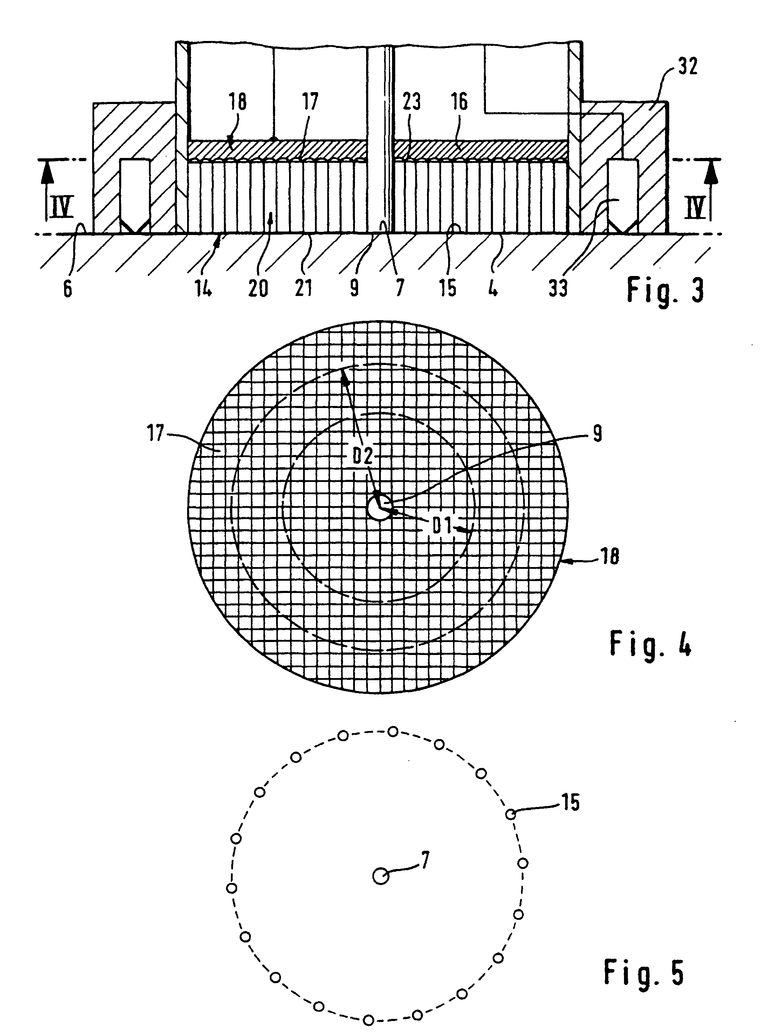

Bei der dargestellten bevorzugten Ausführungsform sind eine Vielzahl von Detektionsmitteln 14 vorgesehen, die es ermöglichen, die Lichtintensität an einer Vielzahl von Detektionsstellen 15 zu bestimmen. Die Detektoren 17 (lichtempfindlichen Elemente) der Detektionsmittel 14 sind an einer Halbleiterschicht 16 ausgebildet, die an entsprechenden Stellen lichtempfindliche Bereiche aufweist. Bevorzugt ist eine regelmäßige Anordnung. Eine solche Einheit ist kommerziell als CCD-Array 18 (insbesondere für Videokameras) erhältlich. Fig. 4 zeigt symbolisch die regelmäßige Anordnung der lichtempfindlichen Teilbereiche 17 eines solchen CCD-Arrays.

Die optische Verbindung zwischen den Detektoren 17 und der jeweils zugeordneten Lichtpassagestelle 21 des Sekundärlichts in der Kontaktfläche 4 wird bei der dargestellten bevorzugten Ausführungsform von einer optischen Faserplatte 20 gebildet, die aus einer Vielzahl von dicht gepackten, senkrecht zu der Kontaktfläche 4 verlaufenden, optischen Fasern besteht. Solche optischen Faserplatten sind kommerziell erhältlich. Ihre Dicke (d.h. Länge der Fasern) beträgt bevorzugt höchstens 5 mm, eine Dicke von etwa 1 bis 2 mm hat sich besonders bewährt. Nähere Einzelheiten können der erwähnten EP-A-0777119 entnommen werden.

Die Fasern geeigneter, optischer Faserplatten haben einen sehr geringen Durchmesser, so daß die optische Verbindung an der einem Detektor 17 zugeordneten Lichtpassagestelle 21 jeweils durch eine Vielzahl von Lichtleitfasern (mehr als 100) gebildet wird. Die in Fig. 3 vertikal zu der Kontaktfläche 4 durch die optische Faserplatte 20 verlaufenden Striche symbolisieren nicht die einzelnen Fasern, sondern die den einzelnen Detektoren 17 zugeordneten Lichtpassagestellen 21 für das Sekundärlicht. Da innerhalb der Faserplatte die Lichtübertragung von Faser zu Faser ("crosstalk") sehr gering ist, werden die Dimensionen der Lichtpassagestellen 21 (und mithin der ihnen entsprechenden Detektionsstellen 15) durch die Begrenzung der lichtempfindlichen Bereiche der Detektoren 17 bestimmt. Ergänzend kann auf mindestens einer Seite der Faserplatte 20 zur Begrenzung der Lichtpassagestellen 21 eine (nicht dargestellte) Maske vorgesehen sein, wie dies in der EP-A-0777119 beschrieben ist.

Die Detektoren 17 sind vorzugsweise fest und unbeweglich (insbesondere durch Kleben oder ein ähnlich dauerhaftes Fixierungsverfahren) mit der detektorseitigen Oberfläche 23 der Faserplatte 20 verbunden. Dabei ist die in den Figuren dargestellte Konstruktion, bei der die Detektoren 17 auf einem gemeinsamen Halbleitersubstrat 18 angeordnet sind, besonders bevorzugt. Insgesamt wird hierdurch eine kompakte Konstruktion mit hoher mechanischer Stabilität ermöglicht. Die mechanische Stabilität trägt auch zur optischen Stabilität bei.

Die Detektoren 17 erzeugen elektrische Meßsignale, die der auf ihre lichtempfindliche Fläche auftreffenden Lichtintensität entsprechen. Sie werden einer Elektronikeinheit 25 zugeleitet. Diese enthält die elektronischen Mittel, um den Lichtsender 8 anzusteuern und aus den von den Detektoren 17 erzeugten Meßsignalen die gewünschte Information über die Polarwinkel-Abhängigkeit des aus der Haut 5 austretenden Sekundärlichts abzuleiten. Außerdem enthält die Elektronikeinheit 25 auch elektronische Mittel zur Ansteuerung der Darstellungsmittel, die zur Darstellung der Vorzugsrichtung bezüglich der Hautoberfläche vorgesehen sind und nachfolgend noch näher erläutert werden. Sie kann deswegen auch als Signalverarbeitungs-, Auswertungs- und Ansteuereinheit bezeichnet werden.

Zur Energieversorgung ist eine Stromversorgungseinheit 26, zweckmäßigerweise mit einer (ladbaren) Batterie, vorhanden. Die Leitungen, die die Elektronikeinheit 25 mit den Detektions- und Darstellungsmitteln verbinden, sind der Übersichtlichkeit halber nur einfach gezeichnet, obwohl sie selbstverständlich entsprechend der Anzahl der zu übertragenden unterschiedlichen Signale n-fach ausgebildet sind.

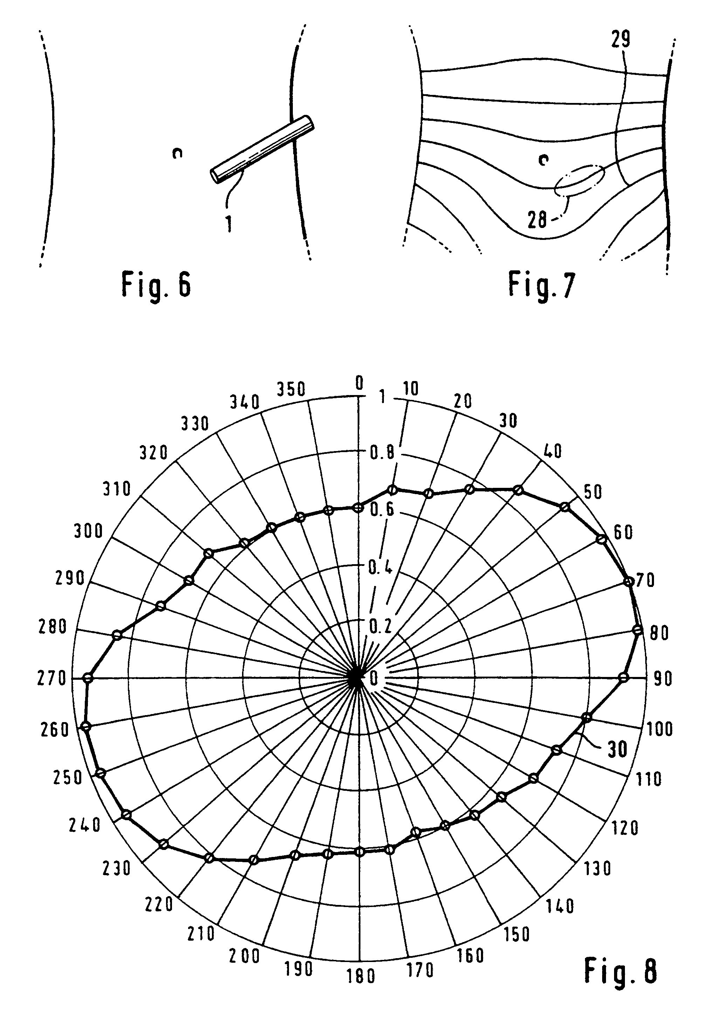

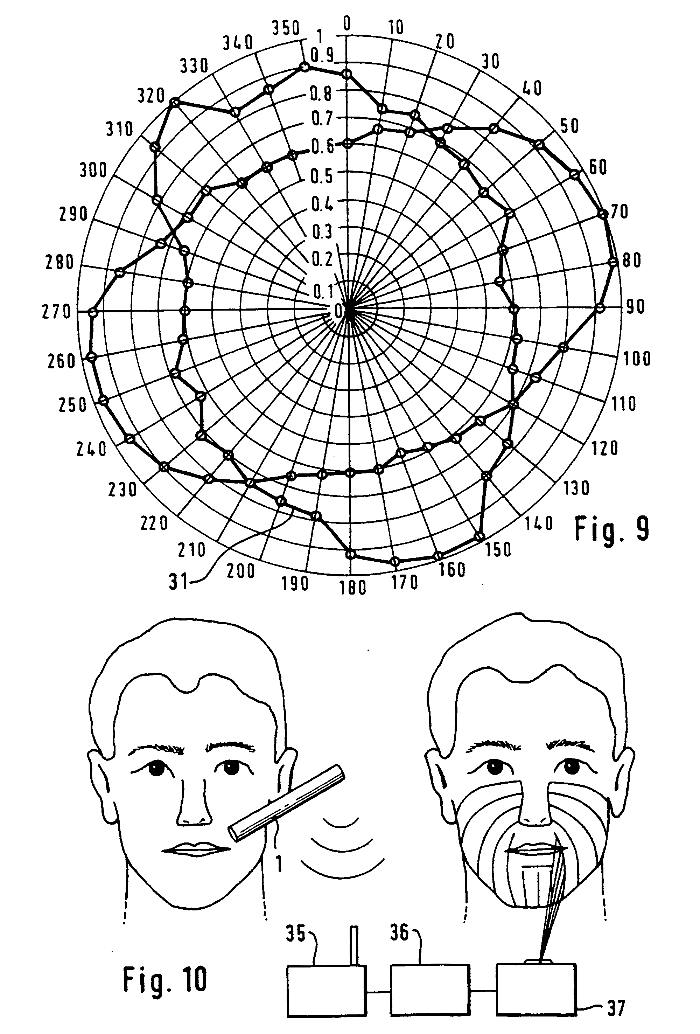

Die Bestimmung einer Polarwinkel-Abhängigkeit der Intensität des aus der Haut 5 austretenden Sekundärlichts mit Hilfe einer Anordnung, wie sie in den Figuren 1 bis 4 dargestellt ist, stellt für den Fachmann kein Problem dar. Wie bereits dargelegt, ist es im allgemeinen vorteilhaft, für eine bestimmte Polarwinkel-Abhängigkeit die Meßsignale von Detektoren 17 zu verwenden, deren zugeordnete Lichtpassagestellen 21 bzw. Detektionsstellen 15 in gleichem Meßabstand zu der Einstrahlungsstelle 7 angeordnet sind, also auf einem Kreis um die Lichtpassagestelle 9 des Primärlichts liegen. In Fig. 4 sind zwei solche Kreise mit den Meßabständen D1 und D2 gestrichelt eingezeichnet. Aus den gemessenen Intensitäten läßt sich unmittelbar die Polarwinkel-Abhängigkeit der Intensität ermitteln und in geeigneter Weise, beispielsweise als Polardiagramm (wie in den Figuren 8 und 9), darstellen.

Die Verwendung eines zweidimensionalen Detektor-Array (wie in Fig. 4 dargestellt) hat den Vorteil, daß eine Vielzahl von Daten über die Verteilung des Sekundärlichts in der Umgebung der Einstrahlungsstelle gewonnen werden. Dadurch ist es möglich, die Polarwinkel-Abhängigkeit für unterschiedliche Meßabstände zwischen Einstrahlungsstelle und Detektionsstelle zu ermitteln. Außerdem können moderne Methoden der Bildverarbeitung oder numerische Algorithmen zur Bestimmung der Vorzugsrichtung eingesetzt werden. Dabei kann es auch zweckmäßig sein, die Polarwinkel-Abhängigkeit für mehrere unterschiedliche Meßabstände zu ermitteln und hieraus die Vorzugsrichtung insgesamt, beispielsweise durch Mittelwertbilung oder einen anderen Algorithmus, abzuleiten.

Da grundsätzlich die Polarwinkel-Abhängigkeit bei einem einzigen Meßabstand ausreicht, kann für die Erfindung jedoch auch eine einfachere Meßapparatur erfolgreich verwendet werden, bei der nur eine verhältnismäßig geringe Zahl von kreisförmig um eine Einstrahlungsstelle 7 angeordneten Detektionssstellen 15 vorgesehen ist. Eine solche Anordnung ist in Fig. 5 dargestellt. Sie läßt sich problemlos realisieren, beispielsweise indem bei einer Anordnung mit dem prinzipiellen Aufbau gemäß den Figuren 1 bis 3 das CCD-Array durch eine kreisförmige Anordnung einzelner Detektoren (z.B. Photodioden, insbesondere Avalanche-Photodioden) ersetzt wird. In diesem Fall kann statt der Faserplatte 20 eine kreisförmige Anordnung einzelner Lichtleitstäbe oder -fasern vorgesehen sein. Es ist auch möglich, die Detektoren unmittelbar in der Nähe der Hautöberfläche an den jeweiligen Detektionsstellen anzuordnen. Um eine gute räumliche Auflösung der Messung der Polarwinkel-Abhängigkeit zu gewährleisten, sollten mindestens neun Detektionsstellen 15 unter unterschiedlichen Polarwinkeln um die Einstrahlungsstelle 7 herum angeordnet sein.

Die Figuren 6 und 7 zeigen symbolisch die Positionierung eines erfindungsgemäßen Geräts 1 in einer Region 28, in der die optimalen Schnittführungslinien 29 in der dargestellten Art und Weise verlaufen. Fig. 8 zeigt ein entsprechendes Polardiagramm für eine Lichtwellenlänge von 800 nm und einem Meßabstand von 6 mm. Dargestellt ist die Intensität in den an dem Null-Grad-Strahl abgetragenen willkürlichen Einheiten, die auf die maximale Intensität normiert wurden. Man erkennt deutlich die ausgeprägte Vorzugsrichtung: Die Lichtintensität in Richtung 70 ° und in die Gegenrichtung (bei 250 °) ist nahezu doppelt so hoch wie die minimale Intensität (etwa in Richtung 160 ° bzw. 340 °). Der Vergleich mit dem in diesem Hautbereich stabilen Verlauf der optimalen Schnittführungslinien zeigt, daß die Abhängigkeit der Lichtintensität von dem Polarwinkel um die Einstrahlungsstelle herum ein zuverlässiges Indiz für die Richtung ist, in der die optimalen Schnittführungslinien verlaufen.

In Fig. 9 sind zwei unterschiedliche Polarwinkel-Abhängigkeitskurven dargestellt, nämlich die anhand der Fig 8 diskutierte Kurve 30 für einen Meßabstand von 6 mm und eine zweite Verteilungskurve 31 für einen Meßabstand von 0,67 mm bei sonst gleichen Bedingungen. Die Kurve 31 zeigte eine abweichende Abhängigkeit der Intensität von dem Polarwinkel mit einer Vorzugsrichtung, die orthogonal zu der Vorzugsrichtung der Kurve 30 liegt. Nach dem gegenwärtigen Kenntnisstand der Erfinder ist dies darauf zurückzuführen, daß bei diesem extrem kurzen Meßabstand Kollagenfaser-Strukturen in der obersten Hautschicht erfaßt werden, die orthogonal zu denen der tieferen Hautschichten verlaufen. Dieser Befund ist somit ein zusätzlicher Beleg für die Präzision des erfindungsgemäßen Verfahrens und für die Möglichkeit, durch Variation des Meßabstands unterschiedliche Schichttiefen in der Haut zu erfassen. Zur Bestimmung der für chirurgische Schnitte entscheidenden Linien-Richtung ist es jedoch bevorzugt, wenn mindestens bei einem Teil der zur Bestimmung der Vorzugsrichtung des diffusen Lichttransports verwendeten Messungen der Meßabstand mindestens 2 mm beträgt. Ein praktikabler Höchstwert des Meßabstands liegt unter etwa 20 mm. Mit anderen Worten sollte der Bereich der Hautoberfläche, in welchem die Polarwinkel-Abhängigkeit des aus der Haut austretenden Sekundärlichts um die Einstrahlungsstelle 7 herum gemessen wird, Abstände von der Einstrahlungsstelle von höchstens 20 mm einschließen.

Im Rahmen der Erfindung wird die Vorzugsrichtung der Lichtintensität und somit der Verlauf der optimalen Schnittführungslinien mittels geeigneter Darstellungsmittel so dargestellt, daß (beispielsweise im Rahmen einer chirurgischen Operation) die Information über die Richtung der optimalen Schnittführungslinien an jeder gewünschten Stelle der Hautoberfläche zuverlässig und problemlos zur Verfügung steht. Hierzu sind zwei grundsätzlich verschiedene Verfahren möglich.

Zum einen können die Darstellungsmittel eine unmittelbar an dem Meßgerät 1 befestigte Markierungseinrichtung 32 aufweisen. Beispielsweise ist bei der Ausführungsform gemäß den Figuren 1 bis 4 um die Hautkontaktplatte herum ein ringförmiger Kranz von Druckelementen 33 vorgesehen, die von der Elektronikeinheit 25 angesteuert werden und mit einem Druckverfahren (beispielsweise Ink-Jet-Druck) eine Markierung auf der Haut erzeugen, die der jeweiligen Vorzugsrichtung entspricht. Der Druckvorgang kann von dem Benutzer jeweils an der gewünschten Stelle der Hautoberfläche ausgelöst werden. Die aufgedruckten Markierungen zeigen den Verlauf der Vorzugsrichtung der Lichtausbreitung und damit der optimalen Schnittführungslinien an.

Für viele Anwendungszwecke ist jedoch die Darstellung der optimalen Schnittführungslinien mittels einer unmittelbar an dem Meßgerät 1 befestigten Markierungseinrichtung nicht ausreichend. Häufig sind nämlich Informationen über größere Abschnitte der Hautoberfläche gewünscht. Dazu muß das Meßgerät 1 über die Haut geführt werden, und es ist erforderlich, eine Darstellung der optimalen Schnittführungslinien in einem größeren Hautareal entweder auf der Haut selbst oder auf einem Bild der Hautoberfläche zu erzeugen.

Die wesentlichen Elemente einer solchen Ausführungsform sind in Fig. 10 schematisch dargestellt. Die jeweilige Position des Meßgeräts 1 wird von einem Positionsdetektor 35 drahtlos bestimmt und an einen Computer 36 übermittelt. Der Positionsdetektor 35 und der Computer 36 bilden insgesamt ein computergesteuertes Positionserfassungssystem. Solche Systeme sind beispielsweise in der Hirnchirurgie gebräuchlich und erlauben eine außerordentlich hohe Genauigkeit der Detektion der Position eines Objekts wie im vorliegenden Fall des Meßgeräts 1.

Zugleich mit der Information über die Position des Meßgeräts 1 wird auch die Information über die jeweilige Richtung der optimalen Schnittführungslinien von dem Meßgerät 1 an den Computer 36 übertragen. Er errechnet den verlauf der optimalen Schnittführungslinien, der dann mit Hilfe geeigneter Darstellungsmittel, beispielsweise eines Laser-Projektors 37, auf der Haut sichtbar gemacht werden kann, wie dies in Fig. 10 dargestellt ist. Alternativ besteht auch die Möglichkeit, beispielsweise durch Laserabtastung, ein dreidimensionales Bild der Hautoberfläche eines individuellen Patienten in einem Computer zu erzeugen und die optimalen Schnittführungslinien auf dem Computerbildschirm zusammen mit einem Bild der Oberfläche so darzustellen, daß der Chirurg oder ein sonstiger Benutzer den Verlauf der optimalen Schnittführungslinien in jeder gewünschten Region der Haut des Patienten erkennen kann.

## Patentansprüche

1. Verfahren zur Detektion der Richtung von Linien einer kosmetisch optimalen chirurgischen Schnittführung in der Haut in-vivo, bei welchem
Licht als Primärlicht an einer punktförmigen Einstrahlungsstelle (7) lokalisiert durch die Hautoberfläche (6) in die Haut (5) eingestrahlt wird,
das Licht in der Haut (5) unter Streuung und Absorption transportiert wird, wobei ein Teil des eingestrahlten Lichts als Sekundärlicht in der Umgebung der Einstrahlungsstelle aus der Hautoberfläche (6) wieder austritt, dadurch charakterisiert, daß
die Polarwinkel-Abhängigkeit einer meßbaren Eigenschaft des aus der Haut (5) austretenden Sekundärlichts um die Einstrahlungsstelle herum gemessen und daraus eine Vorzugsrichtung des diffusen Lichttransports in der Haut (5) bestimmt wird und
die Richtung der Schnittführungslinien aus der Vorzugsrichtung des Lichttransports ermittelt wird.

2. Verfahren nach Anspruch 1, bei welchem der Durchmesser der Einstrahlungsstelle (7) kleiner als 1 mm, bevorzugt kleiner als 0,5 mm ist.

3. Verfahren nach Anspruch 1 oder 2, bei welchem die Polarwinkel-Abhängigkeit durch Messung der meßbaren Eigenschaft an mindestens zwei Detektionsstellen, die einen Polarwinkel-Abstand von mehr als 35° zueinander haben, gemessen wird.

4. Verfahren nach Anspruch 3, bei welchem die Polarwinkel-Abhängigkeit durch Messung der meßbaren Eigenschaft an mindestens drei Detektionsstellen gemessen wird, von denen jeweils zwei einen Polarwinkel-Abstand von höchstens 20° zueinander haben.

5. Verfahren nach einem der vorhergehenden Ansprüche, bei welchem mehrere Detektionsstellen (15) in unterschiedlichen Abständen von der Einstrahlungsstelle (7) angeordnet sind.

6. Verfahren nach einem der vorhergehenden Ansprüche, bei welchem der Bereich, in welchem die Polarwinkel-Abhängigkeit des aus der Haut austretenden Sekundärlichts um die Einstrahlungsstelle (7) herum gemessen wird, Abstände von der Einstrahlungsstelle von höchstens 20 mm einschließt.

7. Verfahren nach einem der vorhergehenden Ansprüche, bei welchem die meßbare Eigenschaft des Sekundärlichts an einer Mehrzahl von in fester räumlicher Zuordnung zu der Einstrahlungsstelle (7) angeordneten Detektionsstellen (15) gemessen wird.

8. Verfahren nach einem der vorhergehenden Ansprüche, bei welchem die Wellenlänge des Primärlichts zwischen 400 und 1400 nm liegt.

9. verfahren nach einem der vorhergehenden Ansprüche, bei welchem die Halbwertsbreite des Primärlichts kleiner als 200 nm ist.

10. Verfahren nach einem der vorhergehenden Ansprüche, bei welchem das Primärlicht polarisiert ist.

11. Verfahren nach einem der vorhergehenden Ansprüche, bei welchem die Polarisation des Sekundärlichts mittels eines in dem Lichtweg des Sekundärlichts angeordneten Polarisationsfilters analysiert wird.

12. Verfahren nach einem der vorhergenden Ansprüche, bei welchem die meßbare Eigenschaft die Intensität des Primärlichts ist.

13. Vorrichtung zur Durchführung des Verfahrens nach einem der vorhergehenden Ansprüche, umfassend
ein Meßgerät (1), welches Einstrahlungsmittel (11), die zum punktförmigen Einstrahlen des Primärlichtes an der Einstrahlungsstelle (7) in die Haut (5) ausgebildet sind, und Detektionsmittel (14), die zur Detektion der Polarwinkel-Abhängigkeit einer meßbaren Eigenschaft des um die Einstrahlungsstelle (7) herum aus der Haut austretenden Sekundärlichtes ausgebildet sind, aufweist, charakterisiert durch
eine Auswerteeinheit (25), die zur Bestimmung einer Vorzugsrichtung des Sekundärlichtes ausgebildet ist und
Darstellungsmittel, die zur Darstellung der Vorzugsrichtung bezüglich der Hautoberfläche ausgebildet sind.

14. Vorrichtung nach Anspruch 13, welche eine optische Faserplatte (20) aufweist, wobei eine Seite der Faserplatte eine Kontaktfläche (4) zum Auflegen auf die Hautoberfläche (6) bildet und die Detektionsmittel (14) auf der anderen Seite der Faserplatte (20) angeordnet sind.

15. Vorrichtung nach Anspruch 13 oder 14, bei welcher die Detektionsmittel (14) ein CCD-Array (18) einschließen.

16. Vorrichtung nach einem der Ansprüche 13 bis 15, bei welcher die Darstellungsmittel eine unmittelbar an dem Meßgerät (1) befestigte Markierungseinrichtung (32) zum Anbringen einer die Vorzugsrichtung anzeigenden Markierung auf der Hautoberfläche aufweisen.

17. Vorrichtung nach einem der Ansprüche 13 bis 16, bei welcher die Darstellungsmittel ein computergesteuertes Positionserfassungssystem (35,36) aufweisen, um die jeweilige Position des Meßgeräts zu bestimmen, und die Darstellungsmittel (37) ein Bild der Linien der optimalen chirurgischen Schnittführung auf der Haut oder auf einer Computerdarstellung der Hautoberfläche (6) erzeugen.

## Claims

1. Method for the in vivo detection of the direction of lines of a cosmetically optimal surgical cut in skin wherein
light is irradiated as primary light into the skin (5) at a point size irradiation site (7) located at the skin surface (6);
the light is scattered and absorbed within the skin (5) and a portion of the irradiated light emerges from the skin surface (6) near the irradiation site as secondary light,
**characterized in that**
the polar angle dependence of a measurable property of the secondary light emerging from the skin (5) is measured in the vicinity of the irradiation site and a preferential direction of diffuse light transport within the skin (5) is determined therefrom; and
the direction of the surgical cut lines is derived from the preferential direction of the light transport.

2. Method according to claim 1, wherein the diameter of the irradiation site (7) is smaller than 1 mm, preferably smaller than 0.5 mm.

3. Method according to any one of claims 1 or 2, wherein the polar angle dependence is determined by measuring the measurable property on at least two detection sites located at a polar angle distance of more than 35°.

4. Method according to claim 3, wherein the polar angle dependence is determined by measuring the measurable property on at least three detection sites, two of which are located at a polar angle distance of at most 20°.

5. Method according to any one of the preceding claims, wherein a plurality of detection sites (15) is located at different distances to the irradiation site (7).

6. Method according to any one of the preceding claims, wherein the area in the vicinity of the irradiated site (7), in which the polar angle dependence of the secondary light emerging from the skin is measured, includes distances from the irradiation site of at most 20 mm.

7. Method according to any one of the preceding claims, wherein the measurable quantity of the secondary light is measured at a plurality of detection sites (15) located in a fixed spatial relationship to the irradiation site (7).

8. Method according to any one of the preceding claims, wherein the wavelength of the primary light is between 400 nm and 1400 nm.

9. Method according to any one of the preceding claims, wherein the wavelength half-width of the primary light is less than 200 nm.

10. Method according to any one of the preceding claims, wherein the primary light is polarized.

11. Method according to any one of the preceding claims, wherein the polarization of the secondary light is analyzed by means of a polarization filter located within the light path of the secondary light.

12. Method according to any one of the preceding claims, wherein the measurable quantity is the intensity of the primary light.

13. Apparatus for performing the method according to any one of the preceding claims comprising:
measurement means (1) with irradiation means (11) for the point irradiation of the primary light into the skin (5) at the irradiation site (7), and detection means (14) for the detection of the polar dependence of a measurable property of the secondary light emerging from the skin in the vicinity of the irradiation site (7);
**characterized in that** it comprises
an evaluation unit (25) for determining the preferential direction of the secondary light, and
display means for displaying the preferential direction relative to the skin surface.

14. Apparatus according to claim 13 comprising an optical fiber plate (20), wherein one side of the fiber plate forms a contact surface (4) for contact with the skin surface (6) and the detection means (14) are located on the opposite surface of the fiber plate (20).

15. Apparatus according to claim 13 or 14, wherein the detection means (14) include a CCD-array (18).

16. Apparatus according to any one of claims 13 to 15, wherein the display means comprise marking means (32), attached to the measurement means (1), to generate a mark on the skin surface which shows the preferential direction.

17. Apparatus according to any one of claims 13 to 16, wherein the display means comprise a computer-controlled position detection system (35,36) to determine the current position of the measurement means, and the display means (37) generate an image of the optimal surgical cut lines on the skin or on a computer image of the skin surface (6).

## Revendications

1. Procédé pour la détection in vivo de la direction de lignes d'une incision chirurgicale esthétiquement optimale dans la peau, dans lequel
- de la lumière, localisée en un lieu d'irradiation ponctuel (7), est irradiée dans la peau (5) au travers de la surface de la peau (6) en tant que lumière primaire,
- la lumière est transportée dans la peau (5) par diffusion et absorption, une partie de la lumière irradiée émergeant de la surface de la peau (6) en tant que lumière secondaire aux alentours du lieu d'irradiation, **caractérisé en ce que**
- la dépendance vis-à-vis de l'angle polaire d'une propriété mesurable de la lumière secondaire émergeant de la peau (5) est mesurée tout autour du lieu d'irradiation et une direction préférentielle du transport de lumière diffus dans la peau (5) est déterminée à partir de là, et
- la direction des lignes d'incision est établie à partir de la direction préférentielle du transport de lumière.

2. Procédé suivant la revendication 1, dans lequel le diamètre du lieu d'irradiation (7) est inférieur à 1 mm, de préférence inférieur à 0,5 mm.

3. Procédé suivant l'une des revendications 1 et 2, dans lequel la dépendance vis-à-vis de l'angle polaire est mesurée par mesure de la propriété mesurable en au moins deux lieux de détection qui ont l'un par rapport à l'autre une distance d'angle polaire de plus de 35°.

4. Procédé suivant la revendication 3, dans lequel la dépendance vis-à-vis de l'angle polaire est mesurée par mesure de la propriété mesurable en au moins trois lieux de détection, ayant deux à deux une distance d'angle polaire de 20° au maximum l'un par rapport à l'autre.

5. Procédé suivant l'une des revendications précédentes, dans lequel plusieurs lieux de détection (15) sont disposés à des distances différentes du lieu d'irradiation (7).

6. Procédé suivant l'une des revendications précédentes, dans lequel la région dans laquelle la dépendance vis-à-vis de l'angle polaire de la lumière secondaire émergeant de la peau est mesurée tout autour du lieu d'irradiation (7) comprend des distances du lieu d'irradiation de 20 mm au maximum.

7. Procédé suivant l'une des revendications précédentes, dans lequel la propriété mesurable de la lumière secondaire est mesurée en une pluralité de lieux de détection (15) disposés en association spatiale fixe avec le lieu d'irradiation (7).

8. Procédé suivant l'une des revendications précédentes, dans lequel la longueur d'onde de la lumière primaire se situe entre 400 et 1400 nm.

9. Procédé suivant l'une des revendications précédentes, dans lequel la largeur de bande à mi-hauteur de la lumière primaire est inférieure à 200 nm.

10. Procédé suivant l'une des revendications précédentes, dans lequel la lumière primaire est polarisée.

11. Procédé suivant l'une des revendications précédentes, dans lequel la polarisation de la lumière secondaire est analysée au moyen d'un filtre de polarisation disposé dans le trajet lumineux de la lumière secondaire.

12. Procédé suivant l'une des revendications précédentes, dans lequel la propriété mesurable est l'intensité de la lumière primaire.

13. Dispositif pour la mise en ceuvre du procédé suivant l'une des revendications précédentes, comprenant
- un appareil de mesure (1) qui présente des moyens d'irradiation (11), réalisés pour l'irradiation ponctuelle de la lumière primaire au lieu d'irradiation (7) dans la peau (5), et des moyens de détection (14), réalisés pour la détection de la dépendance vis-à-vis de l'angle polaire d'une propriété mesurable de la lumière secondaire émergeant de la peau tout autour du lieu d'irradiation (7), **caractérisé par**
- une unité d'analyse (25), réalisée pour la détermination d'une direction préférentielle de la lumière secondaire, et
- des moyens de représentation, réalisés pour représenter la direction préférentielle par rapport à la surface de la peau.

14. Dispositif suivant la revendication 13, qui présente un panneau de fibres optique (20), un côté du panneau de fibres formant une surface de contact (4) pour l'application sur la surface de la peau (6) et les moyens de détection (14) étant disposés sur l'autre côté du panneau de fibres (20).

15. Dispositif suivant l'une des revendications 13 et 14, dans lequel les moyens de détection (14) renferment un tableau à CCD (18).

16. Dispositif suivant l'une des revendications 13 à 15, dans lequel les moyens de représentation présentent un équipement de marquage (32) fixé directement sur l'appareil de mesure (1) pour l'application sur la surface de la peau d'un marquage indiquant la direction préférentielle.

17. Dispositif suivant l'une des revendications 13 à 16, dans lequel les moyens de représentation présentent un système de détection de position (35, 36) piloté par ordinateur pour déterminer la position présente de l'appareil de mesure, et les moyens de représentation (37) produisent une image des lignes d'incision chirurgicale optimale sur la peau ou sur une représentation informatique de la surface de la peau (6).
